# DEMANDE DE BREVET EUROPEEN

(11) **EP 2 011 794 A2**
(43) Date de publication de la demande: **07.01.2009**
(21) Numéro de dépôt: 08150298.1
(22) Date de dépôt: 02.09.2002
(51) Int. Cl.: C07D 401/06, C07D 213/55

(54) **Procédé de synthèse de 5-(3-pyridylméthylène)-imidazolidine-2,4-dione**

(30) Priorité: 05.09.2001 FR 0111637
(62) Demande divisionnaire de: 02774562.9
(71) Demandeur: SOLVAY (Société Anonyme), 1050 Bruxelles (BE)
(72) Inventeur: Callens, Roland, 1850, GRIMBERGEN (BE)
(74) Mandataire: Mross, Stefan P.M.

(57) **Abrégé**

Procédé de synthèse de 5-(3-pyridylméthyl)-imidazolidine-2,4-dione selon lequel on soumet du 5-(3-pyridylméthylène)-imidazolidine-2,4-dione à une réaction d'hydrogénation.

## Description

La présente invention concerne un procédé de synthèse de 5-(3 pyridylméthyl)- imidazolidine- 2,4-dione. Le 5-(3-pyridylméthyl)-imidazolidine-2,4-dione est un produit intermédiaire utilisable pour la synthèse de (3-pyridyl)-alanines, en particulier la D-(3-pyridyl)-alanine. L'invention concerne dès lors aussi un procédé pour la synthèse de (3-pyridyl)-alanine. La D-(3-pyridyl)-alanine peut être utilisée comme constituant de peptides, en particulier dans des antagonistes de LHRH.

La demande de brevet EP-1083170-A1 stipule que la D-(3-pyridyl)-alanine peut être obtenue par réaction enzymatique de 5-(3-pyridylméthyl)-imidazolidine-2,4-dione avec des D-hydantoïnases. Toutefois, cette référence ne décrit pas la synthèse de 5-(3-pyridylméthyl)-imidazolidine-2,4-dione.

Il était dès lors souhaitable de mettre à disposition du 5-(3-pyridylméthyl)-imidazolidine-2,4-dione. Il était particulièrement désirable de disposer d'un procédé efficace et industrialisable de synthèse du 5-(3-pyridylméthyl)-imidazolidine-2,4-dione. Il était encore souhaitable de disposer d'un procédé efficace et industrialisable de synthèse de (3-pyridyl)-alanines, en particulier la D-(3-pyridyl)-alanine.

L'invention concerne dès lors un procédé de synthèse de DL-5-(3-pyridylméthyl)-imidazolidine-2,4-dione selon lequel on soumet du 5-(3-pyridylméthylène)-imidazolidine-2,4-dione à une réaction d'hydrogénation.

L'invention concerne aussi le DL-5-(3-pyridylméthyl)-imidazolidine-2,4-dione.

Il a été trouvé, de manière surprenante, que le procédé selon l'invention permet de convertir, de façon sélective et avec un rendement élevé le 5-(3-pyridylméthylène)- imidazolidine- 2,4-dione en 5-(3-pyridylméthyl)-imidazolidine-2,4-dione. Il est en particulier possible de limiter, voire éviter, la saturation du système aromatique du cycle pyridinique. Le procédé selon l'invention est utilisable pour la fabrication de quantités industrielles de 5-(3--pyridylméthyl)-imidazolidine-2,4-dione pouvant être utilisés pour la fabrication de (3-pyridyl)-alanines, en particulier de D-(3-pyridyl)-alanine.

Dans le procédé selon l'invention, la réaction d'hydrogénation est de préférence effectuée en phase liquide. Dans ce cas, on dissout avantageusement le 5-(3-pyridylméthylène)-imidazolidine-2,4-dione dans un solvant. Des solvants utilisables dans la réaction d'hydrogénation sont choisis, par exemple, parmi des solvants polaires. Conviennent bien des solvants comprenant au moins un groupement OH. Un solvant choisi parmi les alcools aliphatiques comprenant de préférence de 1 à 3 atomes de carbone, l'eau, des solutions aqueuses d'acides, de préférence minéraux, et leurs mélanges a donné de bons résultats. Un système de solvant particulièrement préféré comprend du méthanol et une solution aqueuse d'acide chlorhydrique.

La réaction d'hydrogénation est généralement effectuée en présence d'un catalyseur d'hydrogénation. Le catalyseur d'hydrogénation est avantageusement choisi parmi les métaux du groupe VIII du Tableau périodique des éléments (IL1PAC 1970). On citera en particulier un catalyseur comprenant au moins un métal choisi parmi le palladium, le platine et le rhodium. Un catalyseur comprenant du palladium est préféré. Il a été trouvé qu'un catalyseur d'hydrogénation choisi parmi les métaux du groupe VIII et en particulier un catalyseur comprenant du palladium permet une hydrogénation particulièrement sélective de la double-liaison non-aromatique dans le 5-(3-pyridylméthylène)-imidazolidine-2,4-dione.

Le catalyseur d'hydrogénation est souvent un catalyseur supporté. Des supports utilisables sont choisis par exemple parmi l'alumine, la silice et le charbon actif. Un catalyseur supporté sur charbon actif donne de bons résultats.

Lorsque le catalyseur d'hydrogénation est un catalyseur supporté comprenant un métal du groupe VIII, la teneur en métal est généralement d'au moins 0,1 % en poids par rapport au poids total du catalyseur. Souvent, la teneur en métal est supérieure ou égale à 1 % en poids. De préférence la teneur en métal est supérieure ou égale à 5 % en poids. La teneur en métal est généralement d'au plus 50 % en poids par rapport au poids total du catalyseur.

De manière tout particulièrement préférée, le catalyseur est du palladium supporté de préférence sur un support tel que décrit ci-avant et présentant de préférence une teneur en métal telle que décrite ci-avant.

Dans la réaction d'hydrogénation, la température de la réaction est généralement d'au moins -10°C. Souvent, la température de la réaction est d'au moins 0°C. De préférence, cette température est d'au moins 20°C. La température de la réaction est généralement d'au plus 100°C. Souvent, la température de la réaction est d'au plus 60°C. De préférence, cette température est d'au plus 50°C. Une température d'au plus 40°C est tout particulièrement préférée.

Dans la réaction d'hydrogénation, la pression de la réaction est généralement d'au moins 1 bar absolu. De préférence, la pression est d'au moins 1,5 bar. La pression de la réaction d'hydrogénation est généralement d'au plus 10 bar absolu. De préférence, la pression est d'au plus 5 bar. De manière particulièrement préférée, elle est d'au plus 3 bar.

Dans le procédé selon l'invention, on utilise de préférence de l'hydrogène à titre de réactif d'hydrogénation. Dans ce cas, les valeurs de pression de la réaction d'hydrogénation mentionnées ci-avant correspondent, en général, à la pression d'hydrogène.

Lorsqu'on utilise de l'hydrogène à titre de réactif d'hydrogénation, le rapport molaire entre l'hydrogène et le 5-(3-pyridylméthylène)-imidazolidine-2,4-dione est généralement supérieur ou égal à 1. Ce rapport est généralement d'au plus 100. De préférence ce rapport est d'au plus 10.

Dans la réaction d'hydrogénation, la concentration du 5-(3--pyridylméthylène)-imidazolidine-2,4-dione dans le milieu réactionnel est généralement d'au moins 5 % en poids par rapport au poids total du milieu réactionnél. Souvent, cette concentration est d'au moins 10 % en poids. De préférence, la concentration est d'au moins 20 % en poids. La concentration du 5-(3-pyridylméthylène)-imidazolidine-2,4-dione dans le milieu réactionnel est généralement d'au plus 50 % en poids par rapport au poids total du milieu réactionnel.

Dans une variante particulièrement préférée du procédé selon l'invention le 5-(3-pyridylméthylène)-imidazolidine-2,4-dione a été obtenu par une réaction de condensation entre le pyridine-3-carbaldéhyde et l'imidazolidine-2,4-dione.

La réaction de condensation entre le pyridine-3-carbaldéhyde et l'imidazolidine-2,4-dione est généralement effectuée en présence d'un solvant, de préférence un solvant organique.

Le cas échéant, la teneur en solvant organique dans le milieu réactionnel de la réaction de condensation est généralement d'au plus 80 % en poids par rapport au poids total du milieu réactionnel. De préférence cette teneur est d'au plus 50 % en poids.

Dans un premier aspect, le solvant dans la réaction de condensation est un milieu aqueux. Dans cet aspect on utilise avantageusement un catalyseur. Ce catalyseur est le plus souvent soluble dans l'eau. Un système catalytique qui donne de bons résultats comprend un acide aminé ou un sel d'acide aminé et un composé alcalin inorganique. La glycine est préférée à titre d'acide aminé. Le composé alcalin inorganique est avantageusement choisi parmi l'hydroxyde de sodium et le carbonate de sodium. Le carbonate de sodium est préféré.

Dans un deuxième aspect, le solvant dans la réaction de condensation comprend un solvant organique polaire. Le solvant organique polaire présente généralement un moment dipolaire supérieur à 2 Debye. Sont utilisables notamment des solvants capables de former des liaisons hydrogène, tels que les polyalcohols, les solvants comprenant au moins un groupement amido ou les sulfones et sulfoxides. Citons à titre d'exemple le N,N-diméthylformamide, le N-méthylpyrrolidone, le tétraméthylènesulfone l'éthylèneglycol, la glycérine et leurs mélanges. Des mélanges de N,N-diméthylformamide avec la glycérine donnent de bons résultats.

Dans un troisième aspect, le solvant dans la réaction de condensation comprend un solvant organique de faible viscosité. La viscosité du solvant organique de faible viscosité est généralement inférieure ou égale à 10 mPa.s à 25°C. Une viscosité inférieure ou égale à 5 mPa.s à 25°C convient bien. Une viscosité inférieure ou égale à 1 mPa.s à 25°C est préférée.

Dans un quatrième aspect, le solvant dans la réaction de condensation comprend un solvant organique de faible polarité. Le moment dipolaire solvant organique de faible polarité est généralement d'au plus 2 Debye. Un moment dipolaire inférieur ou égal à 1.8 Debye convient bien. Un moment dipolaire inférieur ou égal à 1 Debye est préféré.

Il a été trouvé de manière surprenante, que la mise en oeuvre d'un solvant organique de faible viscosité et/ou de faible polarité dans la réaction de condensation, permet l'obtention d'un rendement élevé en 5 (3 pyridylméthylène)-imidazolidine-2,4-dione dans des conditions de mise en oeuvre aisées, utilisables pour une synthèse industrielle du 5-(3-pyridylméthylène)-imidazolidine-2,4-dione.

Des solvants organiques particulièrement intéressants à titre de solvant organique de faible viscosité et/ou polarité sont choisis parmi les dialkyléthers linéaires, branchés ou cycliques, les esters carboxyliques et les composés aromatiques. Citons à titre d'exemple la tétrahydrofuranne, l'acétate d'éthyle, le benzène, le toluène et les xylènes. Le toluène donne de bons résultats.

La réaction de condensation entre le pyridine-3-carbaldéhyde et l'imidazolidine-2,4-dione est effectuée, de préférence, en présence d'un catalyseur de condensation. Des catalyseurs de condensation utilisables sont, par exemple, des bases telles qu'en particulier des bases azotées ou leurs sels. Souvent, le catalyseur est une base azotée organique par exemple choisie parmi les alkylamines primaires, secondaires ou tertiaires. Des amines cycliques secondaires ou leurs sels donnent de bons résultats. Citons, à titre d'exemple les dérivés de la morpholine, de la pyrrolidine et de la pipéridine. Parmi ces catalyseurs, un sel de pipéridinium est préféré. L'acétate de pipéridinium convient particulièrement bien à titre de catalyseur de condensation.

La température de la réaction de condensation entre le pyridine-3-carbaldéhyde et l'imidazolidine-2,4-dione est généralement d'au moins 50°C. De préférence la température est d'au moins 60°C. La température de la réaction de condensation entre le pyridine-3-carbaldéhyde et l'imidazolidine-2,4-dione est généralement d'au plus 200°C. De préférence la température est d'au plus 150°C. Une température d'au plus 130°C est plus particulièrement préférée.

Dans le procédé selon l'invention la réaction de condensation est généralement effectuée à un pH d'au moins 7. Le pH est de préférence d'au moins 8. Dans le procédé selon l'invention la réaction de condensation est généralement effectuée à un pH d'au plus 12. Le pH est de préférence d'au plus 10.

L'invention concerne aussi le procédé de fabrication de 5-(3--pyridylméthylène)-imidazolidine-2,4-dione par réaction de condensation entre le pyridine-3-carbaldéhyde et l'imidazolidine-2,4-dione décrit ci-avant.

L'invention concerne aussi un procédé de synthèse de (3-pyridyl)-alanine selon lequel on soumet du 5-(3-pyridylméthyl)-imidazolidine-2,4-dione obtenu selon le procédé selon l'invention à une réaction d'hydrolyse suivie d'une réaction de décarbamatation.

La réaction d'hydrolyse peut être effectuée par exemple par réaction avec une base à un pH supérieur à 12.

De préférence, l'hydrolyse est effectuée par réaction avec une hydantoïnase, en particulier une D-hydantoïnase. Dans ce cas, la (3-pyridyl)-alanine obtenue est la D-(3-pyridyl)-alanine. Des informations concernant cette réaction sont contenues dans la demande de brevet EP1083170-A1 citée plus haut.

La décarbamatation peut être effectuée, par exemple, par nitrosation.

La (3-pyridyl)-alanine, en particulier la D-(3-pyridyl)-alanine obtenu selon le procédé selon l'invention, peut être utilisée pour la fabrication d'un peptide. Des techniques conventionnelles de couplage peptidique sont utilisables pour la fabrication du peptide.

Les exemples ci-après entendent illustrer l'invention sans toutefois la limiter.

### Exemple 1 - Synthèse de 5-(3-pyridylméthylène)-imidazolidine-2,4-dione

On a introduit 0,1 mole d'imidazolidine-2,4-dione, 3 ml d'acide acétique glacial, 0,12 mole de pyridine-3-carbaldéhyde et 30 ml de toluène dans un ballon. A ce mélange, on a rajouté 5 ml de pipéridine. L'ensemble a été chauffé au reflux pendant 17 h. Après refroidissement on a ajouté de l'isopropanol. Le précipité obtenu a été filtré et lavé à l'isopropanol. Le rendement en 5-(3 pyridylméthylène)-imidazolidine-2,4-dione était de 85 %.

Le produit obtenu était directement utilisable pour la mise en oeuvre dans la réaction d'hydrogénation sans épuration supplémentaire.

### Exemple 2 - Synthèse de DL-5-(3-pyridylméthyl)-imidazolidine-2,4-dione

On a introduit de 0,13 mole de 5-(3-pyridylméthylène)-imidazolidine-2,4-dione obtenu conformément au procédé de l'exemple 1, 250 ml d'acide chlorhydrique IN, 200 ml de méthanol et 3,26 g d'un catalyseur constitué de palladium sur charbon actif (concentration en Pd 10 % en poids). On a effectué la réaction d'hydrogénation à température ambiante sous une pression initiale d'hydrogène de 2,1 bar pendant 15 h. Le milieu réactionnel a été filtré et le DL-5-(3-pyridylméthyl)-imidazolidine-2,4-dione brut obtenu après évaporation a été cristallisé avec un mélange 1 :1 de méthanol :éther de méthyle tert.butylique.

### Rendement 79 %

Le DL-5-(3-pyridylméthyl)-imidazolidine-2,4-dione obtenu était directement utilisable pour la mise en oeuvre dans une réaction d'hydrolyse sans épuration supplémentaire. Il était en particulier adapté pour la mise en oeuvre dans une réaction d'enzymolyse. Le procédé selon l'invention permet d'accéder au DL-5-(3-pyridylméthyl)-imidazolidine-2,4-dione de manière simple et industrialisable et avec un rendement élévé.

## Revendications

1. - Procédé de synthèse de 5-(3-pyridylméthyl)-imidazolidine-2,4-dione selon lequel on soumet du 5-(3-pyridylméthylène)-imidazolidine-2,4-dione à une réaction d'hydrogénation.

2. - Procédé selon la revendication 1, dans lequel la réaction d'hydrogénation est effectuée en phase liquide.

3. - Procédé selon la revendication 1 ou 2, dans lequel la réaction est effectuée en présence d'un catalyseur d'hydrogénation.

4. - Procédé selon la revendication 3, dans lequel le catalyseur d'hydrogénation est choisi parmi les métaux du groupe VIII du Tableau périodique des éléments.

5. - Procédé selon la revendication 4, dans lequel le catalyseur est du palladium supporté.

6. - Procédé selon l'une quelconque des revendications 1 à 5, dans lequel la température de la réaction est de -10°C à +100°C.

7. - Procédé selon l'une quelconque des revendications 1 à 6, dans lequel la pression de la réaction est de 1 bar à 15 bar.

8. - Procédé selon l'une quelconque des revendications 1 à 7, dans lequel on utilise de l'hydrogène à titre de réactif d'hydrogénation.

9. - Procédé selon la revendication 8, dans lequel le rapport molaire entre l'hydrogène et le 5-(3-pyridylméthylène)-imidazolidine-2,4-dione est de 1 à 1000.

10. - Procédé selon l'une quelconque des revendications 1 à 9, dans lequel la concentration du 5-(3-pyridylméthylène)-imidazolidine-2,4-dione dans le milieu réactionnel est de 5 % en poids à 50 % en poids par rapport au poids total du milieu réactionnel.

11. - Procédé de synthèse de (3-pyridyl)-alanine dans lequel
(a) on fabrique du 5-(3-pyridylméthyl)-imidazolidine-2,4-dione selon le procédé d'une quelconque des revendications 1 à 10
(b) on soumet du 5-(3-pyridylméthyl)-imidazolidine-2,4-dione obtenu à une réaction d'hydrolyse suivie d'une réaction de décarbamatation.

12. - Procédé selon la revendication 11, dans lequel la (3-pyridyl)-alanine obtenue est la D-(3-pyridyl)-alanine.
